# EUROPEAN PATENT APPLICATION

(11) **EP 4 163 291 A1**
(43) Date of publication of application: **12.04.2023**
(21) Application number: 20939771.0
(22) Date of filing: 01.12.2020
(51) Int. Cl.: C07K 14/165, A61K 39/215, A61P 31/14, A61P 37/04, A61K 31/522, A61K 39/39, A61K 47/55, A61K 47/65

(54) **POLYPEPTIDE VACCINE COUPLED WITH TLR7 AGONIST FOR NOVEL CORONAVIRUS AND USE THEREOF**

(30) Priority: 08.06.2020 CN 202010514683
(71) Applicant: Shanghai Institute of Materia Medica, Chinese Academy of Sciences, Pudong, Shanghai 201203 (CN); Shenzhen University, Shenzhen, Guandong 518061 (CN)
(72) Inventor: GONG, Likun, Shanghai 201203 (CN); REN, Jin, Shanghai 201203 (CN); JIN, Guangyi, Shenzhen, Guangdong 518061 (CN); HUANG, Wei, Shanghai 201203 (CN); QIN, Qiuping, Shanghai 201203 (CN); LONG, Yiru, Shanghai 201203 (CN); SUN, Jianhua, Shanghai 201203 (CN); LIU, Tingting, Shanghai 201203 (CN); TANG, Feng, Shanghai 201203 (CN); ZHU, Peng, Shanghai 201203 (CN)
(74) Representative: karo IP
(86) International application number: PCT/CN2020/133168
(87) International publication number: WO 2021/248853

(57) **Abstract**

Disclosed are a polypeptide vaccine coupled with a TLR7 agonist for novel coronavirus and the use thereof. Specifically, the present invention provides a vaccine polypeptide for novel coronavirus pneumonia based on the basis of the analytical study of the RBD sequence and structural information of the S protein of SARS-CoV-2, wherein the vaccine polypeptide has the following structural formula: Z-(J-U)n, where in the formula, Z, J, U, n, etc. are as defined in the description. Also provided in the present invention are a vaccine composition containing the vaccine polypeptide and the use thereof.

## Description

### TECHNICAL FIELD

The present invention relates to the fields of polypeptide medicines and polypeptide vaccines, in particular to polypeptide vaccine coupled with TLR7 agonist for novel coronavirus and use thereof.

### BACKGROUND

The novel coronavirus pneumonia (Corona virus disease 2019, COVID-19) caused by the coronavirus SARS-CoV-2 is extremely contagious and has caused severe epidemics worldwide, endangering the lives of millions of people. However, there is still a lack of clearly effective drugs and measures for prevention and treatment of COVID-19, and supportive treatment and symptomatic treatment are the main focus clinically.

Establishing herd immunity against SARS-CoV-2 through vaccines is the ultimate way to control and block the COVID-19 epidemic. At present, many types of COVID-19 vaccines have been in preclinical or clinical trials, including live attenuated vaccines, inactivated virus vaccines, recombinant virus vector vaccines, recombinant protein vaccines, DNA vaccines, RNA vaccines and peptide vaccines, etc..

However, the protective effect of vaccines developed at present is limited, for in presence of problem such as low immunogenicity or virus immune escape and so on.

Therefore, there is an urgent need in this field to develop new vaccines that efficiently stimulate the human body to produce an immune response against SARS-CoV-2, in order to produce blocking anti-SARS-CoV-2 antibodies in the vaccinators, thereby providing powerful immune protection.

### SUMMARY OF THE INVENTION

The purpose of the present invention is to provide a new vaccine that efficiently stimulates the human body to produce an immune response against SARS-CoV-2, and then produce blocking anti-SARS-CoV-2 antibodies in vaccinators, thereby providing powerful immune protection.

The first aspect of the present invention provides a vaccine polypeptide of novel coronavirus, comprising an antigenic polypeptide and optionally a TLR7 agonist coupled to the antigenic polypeptide.

In another preferred embodiment, the vaccine polypeptide has a structure of formula V or an oligomer comprising the structure of formula V:

Z-(U)n (V)

wherein,
Z is an antigenic polypeptide that has at least one T cell epitope and/or at least one B cell epitope of the novel coronavirus S protein; and, the antigenic polypeptide has an amino acid sequence derived from the RBM region of the S protein;
each U is independently a TLR7 agonist;
n is 0 or a positive integer; and
"-" is a chemical bond or linker or connector.

In another preferred embodiment, the vaccine polypeptide has a structure of Formula I or an oligomer comprising a structure of Formula I:

Z-(J-U)n (I)

wherein,
Z is an antigenic polypeptide that has at least one T cell epitope and/or at least one B cell epitope of the novel coronavirus S protein; and, the antigenic polypeptide has an amino acid sequence derived from the RBM region of the S protein;
each U is independently a TLR7 agonist;
n is 0 or a positive integer; and
J is a chemical bond or linker.

In another preferred embodiment, the vaccine polypeptide can stimulate primates and rodents to produce neutralizing antibodies that block the binding of RBD to ACE2.

In another preferred embodiment, the vaccine polypeptide can stimulate primates to produce cellular immunity and humoral immunity.

In another preferred embodiment, the primates include humans and non-human primates.

In another preferred embodiment, the length of the antigenic polypeptide is 8-100 amino acids, preferably 10-80 amino acids.

In another preferred embodiment, the antigenic polypeptide has an amino acid sequence derived from the RBD region of the novel coronavirus S protein.

In another preferred embodiment, the antigenic polypeptide has an amino acid sequence derived from the RBM region of the RBD region.

In another preferred embodiment, the RBM region refers to amino acids positions 438-506 of the novel coronavirus RBD protein.

In another preferred embodiment, the antigenic polypeptide "having an amino acid sequence derived from the RBM region of the RBD protein" means that the amino acid sequence of the antigenic polypeptide has homology (or identity) with the RBM region, and the the homology is ≥80%, preferably ≥85%, more preferably >_90%, and most preferably >_95%.

In another preferred embodiment, the antigenic polypeptide competitively binds to the human ACE2 protein with the S protein of the novel coronavirus.

In another preferred embodiment, the "competitive binding" means that the antigenic polypeptide and the S protein of the novel coronavirus bind to the same or substantially the same binding domain (or amino acid segment) of the human ACE2 protein.

In another preferred embodiment, the antigenic polypeptide and the S protein of the novel coronavirus bind to the same binding segment on the human ACE2 protein.

In another preferred embodiment, the competitive binding includes blocking or non-blocking competitive binding.

In another preferred embodiment, the antigenic polypeptide is artificially synthesized or recombinant antigenic polypeptide.

In another preferred embodiment, the antigenic polypeptide is selected from the group consisting of:
(a) a pollypeptide having any one of the amino acid sequence shown in SEQ ID No: 1-12;
(b) a derivative polypeptide formed by one or more amino acids addition, one or more amino acids substitution, or 1-3 amino acids deletion to the amino acid sequence of the polypeptide in (a), and the derivative polypeptide has the same function as the original polypeptide before derivatization.

In another preferred embodiment, the "substantially the same function" means that the derivative polypeptide has substantially the same immunogenicity for stimulating immune response, ability for competitive binding to the human ACE2 protein with the S protein of the novel coronavirus, and/or has at least one T cell epitope, and/or has at least one B cell epitope.

In another preferred embodiment, the structure of the antigenic polypeptide is as shown in formula II:

X1-X-X2 (II),

wherein,
(a) X is a core fragment, wherein the sequence of the core fragment is selected from one or more of SEQ ID NO:1-12 (see Table A);
(b) each X1 and X2 is independently none, 1, 2, or 3 amino acids, and the total number of amino acids of X1 and X2 is <_ 4, preferably 3, 2, 1, and more preferably 0 or 1; and
(c) "-" represents peptide bond, peptide linker, or other linker (that is, X1 and X and/or X and X2 are connected by peptide bonds, peptide linkers (such as a flexible linker consisting of 1-15 amino acids) or other linkers).

In another preferred embodiment, the antigenic polypeptide is selected from Table A:

**Table A Antigenic peptides**

| Peptide ID | Sequence | Length (aa) | SEQ ID No: |
|---|---|---|---|
| P-33 | YNYLYRLFRKSNLKPFERDISTEIY | 25aa | 1 |
| P-37 | YRLFRKSNLKPFERDISTEIYQAGS | 25aa | 2 |
| P-41 | KRSFIEDLLFNKVTLADAGFIKQYG | 25aa | 3 |
| P-67 | YAWNRKRISNCVADYSVLYNSASFSTF K | 28aa | 4 |
| P-67-F1 | CYAWNRKRISN | 11aa | 5 |
| P-67-F2 | CVADYSVLYNSASFSTFK | 18aa | 6 |
| P-71 | GVEGFNCYFPLQSYGFQPTNGVGYQP YR | 28aa | 7 |
| P-73 | FQPTNGVGYQPYRVVVLSFELLHAPAT V | 28aa | 8 |
| P-79 | ISNCVADYSVLYNSASFSTFKC | 22aa | 9 |
| P-85 | DYSVLYNSASFSTFKCYGVSPT | 22aa | 10 |
| P-86 | TEIYQAGSTPCNGVEGFNCYFP | 22aa | 11 |
| LY54 | LFRKSNLKPFERDISTEIYQAGSTPCNG VEGFNCYFPLQSYGFQPTNGVGYQPY | 54aa | 12 |

In another preferred embodiment, each X1 and X2 is independently none, K, C, G, L, or A.

In another preferred embodiment, X1 is none, K, or C.

In another preferred embodiment, X2 is none, K, or C.

In another preferred embodiment, the antigenic polypeptide has at least one T cell epitope and/or at least one B cell epitope of the RBD region of the novel coronavirus S protein.

In another preferred embodiment, the antigenic polypeptide has at least one T cell epitope and/or at least one B cell epitope of the RBM region of the novel coronavirus S protein.

In another preferred embodiment, the antigenic polypeptide has at least one T cell epitope, preferably 1, 2, 3 or 4 T cell epitopes, more preferably 1 or 2 T cell epitopes.

In another preferred embodiment, the antigenic polypeptide has at least one B cell epitope, preferably 1, 2, 3 or 4 B cell epitopes, more preferably 1 or 2 B cell epitopes.

In another preferred embodiment, the antigenic polypeptide has 1-2 T cell epitopes and 0-2 B cell epitopes, preferably 1-2 T cell epitopes and 0-1 B cell epitopes.

In another preferred embodiment, the molecule number n of the TLR agonist is 1, 2, 3, 4, 5 or 6; preferably 1, 2, 3 or 4.

In another preferred embodiment, the TLR7 agonist is a small molecule agonist.

In another preferred embodiment, the TLR7 agonist includes: SZU-101:

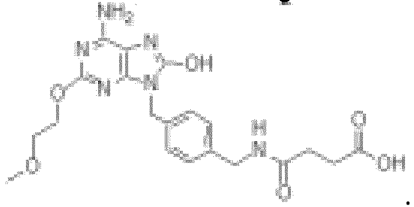

In another preferred embodiment, the TLR7 agonist (such as SZU-101) is attached to the terminal amino group or side chain amino group of the antigenic polypeptide.

In another preferred embodiment, the TLR7 agonist (such as SZU-101) is attached to the sulfhydryl group of the antigenic polypeptide.

In another preferred embodiment, the SZU-101 is attached to the amino group of the antigenic polypeptide and forms the structure shown in S 1: 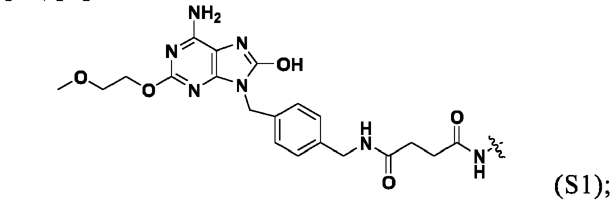 or
the SZU-101 is attached to the sulfhydryl group of the antigenic polypeptide and forms the structure shown in S2:

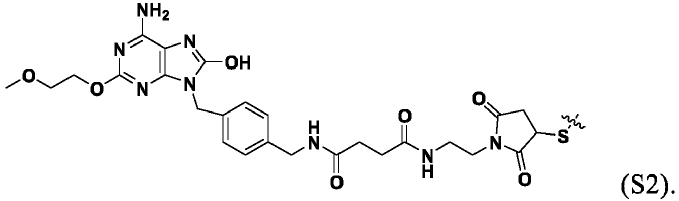

In another preferred embodiment, the TLR7 agonist is site-specifically and/or randomly attached to the antigenic polypeptide (ie, in Formula I, the U is site-specifically and/or randomly attached to Z).

In another preferred embodiment, the U is site-specifically attached to Z.

In another preferred embodiment, the U is site-specifically attached to amino acid sites of antigenic polypeptide Z selected from the group consisting of G, K, L, A, C, and combinations thereof.

In another preferred embodiment, the vaccine polypeptide is selected from the group of coupled peptides consisting of:
The vaccine polypeptide is selected from the group of coupled peptides consisting of:
(S1)-GVEGFNCYFPLQSYGFQPTNGVGYQPYRK-(S1) (SEQ ID No: 14)
wherein, SZU-101 is attached to the N-terminal amino group of G and the side chain amino group of K in the amino acid sequence through the S1 structure;
   (S1)₂-KGVEGFNCYFPLQSYGFQPTNGVGYQPYR (SEQ ID No: 15)
wherein, SZU-101 is attached to the N-terminal and side chain amino group of K in the amino acid sequence through the S1 structure; 
(S1)-LFRK(-S1)SNLK(-S1)PFERDISTEIYQAGSTPCNGVEGFNCYFPLQSY
GFQPTNGVGYQPY(SEQ ID No: 12)
wherein, SZU-101 is attached to the N-terminal amino group of L and the side chain amino group of K in the amino acid sequence through the S1 structure;
   GVEGFNC(-S2)YFPLQSYGFQPTNGVGYQPYRK (SEQ ID No: 14)
wherein, SZU-101 is attached to the sulfhydryl group of C in the amino acid sequence through the S2 structure;
   (S2)-CYRLFRKSNLKPFERDISTEIYQAGS (SEQ ID No: 16)
wherein, SZU-101 is attached to the sulfhydryl group of C in the polypeptide through the S2 structure;
   (S2)-CYAWNRKRISN (SEQ ID No: 5)
wherein, SZU-101 is attached to the sulfhydryl group of C in the polypeptide through the S2 structure; and
   (S2)-CVADYSVLYNSASFSTFK (SEQ ID No: 6)
wherein, SZU-101 is attached to the sulfhydryl group of C in the polypeptide through the S2 structure;
and the structure of S1 and S2 is as defined above.

In another preferred embodiment, the coupled peptide is selected from Table B:

**Table B Coupled peptides**

| Coupled peptide | Structural formula | X1 | X0 | X2 |
|---|---|---|---|---|
| P-71-C-S1 | (S1)-GVEGFNCYFPLQSYGFQPTNG VGYQPYRK-(S1) | None | SEQ ID No: 7 | K |
| P-71-N-S1 | (S1)₂-KGVEGFNCYFPLQSYGFQPT NGVGYQPYR | K | SEQ ID No: 7 | None |
| LY54-S1 | (S1)-LFRK(-S1)SNLK(-S1)PFERDIS TEIYQAGSTPCNGVEGFNCYFPLQ SYGFQPTNGVGYQPY | None | SEQ ID No: 12 | None |
| P-71-S2 | GVEGFNC(-S2)YFPLQSYGFQPTNG VGYQPYRK | None | SEQ ID No: 7 | K |
| P-37-S2 | (S2)-CYRLFRKSNLKPFERDISTEIY QAGS | C | SEQ ID No: 2 | None |
| P-67-F1-S2 | (S2)-CYAWNRKRISN | None | SEQ ID No: 5 | None |
| P-67-F2-S2 | (S2)-CVADYSVLYNSASFSTFK | None | SEQ ID No: 6 | None |

Note: In the table, S1 and S2 represent the above-mentioned connection structure of SZU-101.

In another preferred embodiment, the vaccine polypeptide is selected from the group consisting of: P-37 or its conjugates with TLR7 agonist, P-67-F1 or its conjugates with TLR7 agonist, P-71 or its conjugates with TLR7 agonist, LV54 or its conjugates with TLR7 agonist, and combinations thereof.

In another preferred embodiment, the TLR7 agonist is SUZ-101.

The second aspect of the present invention provides a separated peptide set, comprising at least two vaccine polypeptides of the novel coronavirus according to the first aspect of the present invention.

In another preferred embodiment, the peptide set comprises at least 2-20 kinds, preferably 2-12 kinds (such as 2, 3, 4, 5, 6, 7, 8, 9, 10, 11, 12 kinds) of the vaccine polypeptide.

The third aspect of the present invention provides a pharmaceutical composition, comprising the vaccine polypeptide of novel coronavirus according to the first aspect of the present invention or the peptide set according to the second aspect of the present invention, and a pharmaceutically acceptable carrier.

In another preferred embodiment, the pharmaceutical composition is a vaccine composition.

In another preferred embodiment, the vaccine composition is monovalent or multivalent.

In another preferred embodiment, the pharmaceutical composition further comprises an adjuvant, and various aluminum adjuvants are preferred. The molar or weight ratio of active peptide and adjuvant (such as aluminum) in the composition is between 1:100, preferably between 1:40 and 1:60.

In another preferred embodiment, the pharmaceutical composition includes single drug, compound drugs, or synergistic drugs.

In another preferred embodiment, the dosage form of the pharmaceutical composition is liquid, solid, or gel.

In another preferred embodiment, the pharmaceutical composition is administered in a manner selected from the group consisting of: subcutaneous injection, intradermal injection, intramuscular injection, intravenous injection, intraperitoneal injection, microneedle injection, oral administration, or oral and nasal spray and atomization inhalation.

The fourth aspect of the present invention provides a use of the novel coronavirus vaccine polypeptide according to the first aspect or the peptide set according to the second aspect or the pharmaceutical composition according to the third aspect of the present invention, in the manufacture of a medicament for the prevention of coronavirus SARS-CoV-2 infection or related disease.

In another preferred embodiment, the coronavirus SARS-CoV-2 related disease is selected from the group consisting of: respiratory infection, pneumonia and its complications, and combinations thereof.

In another preferred embodiment, the coronavirus SARS-CoV-2 related disease is novel coronavirus pneumonia (COVID-19).

The fifth aspect of the present invention provides a cell preparation, comprising (a) immune cells immuno-activiated by the novel coronavirus vaccine polypeptide according to the first aspect of the present invention or the peptide set according to the second aspect of the present invention; and (b) a pharmaceutically acceptable carrier.

In another preferred embodiment, the immune cells are selected from the group consisting of: dendritic cells, natural killer cells NK, lymphocytes, monocytes/macrophages, granulocytes, and combinations thereof.

In another preferred embodiment, the activation is *in vitro* activation.

In another preferred embodiment, the *in vitro* activation comprises: culturing the immune cells for a period of time (such as 6-48 hours) in the presence of the vaccine polypeptide to obtain the immuno-activiated immune cells.

In another preferred embodiment, the cell preparation is a liquid preparation comprising living cells.

In another preferred embodiment, the cell preparation is reinfused through intravenous administration.

The sixth aspect of the present invention provides a method for generating an immune response against the coronavirus SARS-CoV-2, which comprises the steps of: administering the novel coronavirus vaccine polypeptide according to the first aspect, the peptide set according to the second aspect or the pharmaceutical composition according to the third aspect of the present invention to a subject in need thereof.

In another preferred embodiment, the subject comprises human or non-human mammals.

In another preferred embodiment, the non-human mammals comprise non-human primates (such as monkeys).

In another preferred embodiment, the method induces the production of neutralizing antibodies against the coronavirus SARS-CoV-2 in the subject.

In another preferred embodiment, the neutralizing antibody blocks the binding of coronavirus SARS-CoV-2 to human ACE2 protein.

The seventh aspect of the present invention provides a fusion protein, including a carrier protein and the vaccine polypeptide according to the first aspect of the present invention fused to the fusion protein.

In another preferred embodiment, the fusion protein is a non-natural protein.

In another preferred embodiment, the fusion protein has a structure of formula IIIa or IIIb:

P1-P2 (IIIa)

P2-P1 (IIIb)

wherein, P1 is the vaccine polypeptide described in the first aspect of the present invention, and P2 is the carrier protein.

In another preferred embodiment, P1 can be a single vaccine polypeptide, or multiple identical or different vaccine polypeptides (or antigenic polypeptides) in series.

In another preferred embodiment, the P1 is coupled to one or more TLR7 agonists.

The eighth aspect of the present invention provides a pharmaceutical composition, comprising (a) the fusion protein according to the seventh aspect of the present invention or immune cells immuno-activated by the fusion protein; and (b) a pharmaceutically acceptable carrier .

The ninth aspect of the present invention provides the use of the fusion protein according to the seventh aspect of the present invention or the pharmaceutical composition according to the eighth aspect of the present invention, in the manufacture of a medicament for the prevention of coronavirus SARS-CoV-2 infection or related disease.

It should be understood that within the scope of the present invention, each technical features of the present invention described above and in the following (as examples) may be combined with each other to form a new or preferred technical solution, which is not listed here due to space limitations.

### DESCRIPTION OF FIGURES

Figure 1 shows the amino acid sequence analysis of SARS-CoV-2 S protein and its key sites of interaction with ACE2 in RBD.
Figure 2 shows the interaction structure and key sites of SARS-CoV-2 S protein RBD region and human ACE2.
Figure 3 shows a comprehensive analysis chart of the possibility of binding of polypeptides P-37, P-67 and P-71 to 24 major HLA class II allele typing molecules in the population.
Figure 4 shows the linear B cell epitope contained in the S protein RBD predicted by the BepiPred software and the relative position relationship with the polypeptides P-37, P-67 and P-71.
Figure 5 shows the structure of the conformational B cell epitope contained in the S protein RBD predicted by the Discotope software.
Figure 6 shows the positions of the polypeptides P-37, P-67, P-71 and LY54 on the RBD structure of the S protein.
Figure 7 shows the chemical structure of the TLR7 small molecule agonist SZU-101.
Figure 8 shows the results of *in vitro* mouse spleen lymphocyte cytokine release induced by polypeptide P-71 and its SZU-101 coupled peptide.
Figure 9 shows the results of *in vitro* mouse splenic lymphocyte proliferation induced by polypeptide P-71 and its SZU-101 coupled peptide.
Figure 10 shows the production of anti-RBD antibody after immunized BALB/c mice by the polypeptides P-37, P-71 and P-67-F2 and their respective SZU-101 coupled peptides (see Table B) individually or in combination.
Figure 11 shows the production of anti-RBD antibodies after LY54 and LY54-S1 immunized cynomolgus monkeys, and the production of anti-RBD antibody after the SZU-101 coupled peptides of polypeptides P-37, P-71 and P-67-F2 (see Table B) mixed immunized cynomolgus monkeys.
Figure 12 shows the anti-RBD antibodies produced by immunizing cynomolgus monkeys with LY54, LY54-S1 and mixed coupled peptides (see Table B), have the neutralizing activity of blocking the binding of RBD to ACE2.
Figure 13 shows polypeptides P-37, P-71 and LY54 have good effects on binding to human ACE2.

### DETAILED DESCRIPTION

Upon extensive and intensive studies, based on the analysis of the sequence and structure of the SARS-CoV-2 S protein RBD, the inventors have analyzed the CD4+T/CD8+T cell epitope, linear/conformational B cell epitope, key sites of interaction, surface features, glycosylation modification sites, and peptide physical and chemical properties of the S protein, screened and identified vaccine polypeptides that can effectively induce the mammalian organism to produce immune response against the coronavirus SARS-CoV-2 for the first time. Experiments have shown that the vaccine polypeptides of the present invention can effectively trigger cellular and humoral immunity against SARS-CoV-2 in rodents (such as mice) and primates (such as cynomolgus monkeys), thereby producing a higher titer neutralizing antibodies that block the binding of RBD to ACE2, so the present invention has potential application prospects in the prevention or treatment of novel coronavirus pneumonia. The present invention has been completed on this basis.

Specifically, based on the analysis of the RBD sequence and structural information of SARS-CoV-2 S protein, by determining the T/B cell epitope of the S protein, and comprehensively considering the structural surface characteristics of the S protein, the key sites of interaction with ACE2 and the physical and chemical properties of the polypeptides, etc., the present invention screened and determined 12 antigenic polypeptides shown in Table A, and the antigenic polypeptides (including P-37, P-67 (including P-67-F1 and P-67-F2) ), P-71 and LY54) were coupled with TLR7 small molecule agonists to form the coupled peptides shown in Table B. Experiments have shown that the coupled polypeptide vaccine may activate stronger cellular and humoral immunity in mice and cynomolgus monkeys, and produce higher-titer neutralizing antibodies that block the binding of RBD to ACE2.

### Term

### Coronavirus SARS-CoV-2

Coronavirus (CoV) belongs to the nidovirales coronaviridae, which is an enveloped positive-strand RNA virus, and its subfamily includes α, β, δ and γ four genera.

Among the currently known coronaviruses that infect humans, HCoV-229E and HCoV-NL63 belong to the α genus coronavirus, and HCoV-OC43, SARS-CoV, HCoV-HKU1, MERS-CoV and SARS-CoV-2 are all β genus coronavirus.

The novel coronavirus (SARS-CoV-2) that broke out at the end of 2019 has about 80% similarity with SARS-CoV and 40% similarity with MERS-CoV. It also belongs to the β genus coronavirus.

The genome of this type of virus is a single positive-stranded RNA, which is one of the largest RNA viruses in the genome, which codes replicase, spike protein, capsule protein, envelope protein, and nucleocapsid protein,etc.. In the initial stage of virus replication, the genome is translated into two peptide chains of several thousand amino acids, i.e the precursor polyprotein, and then the precursor protein is cleaved by proteases to produce non-structural proteins (such as RNA polymerase and helicase) and structural proteins (such as spike proteins) and accessory proteins.

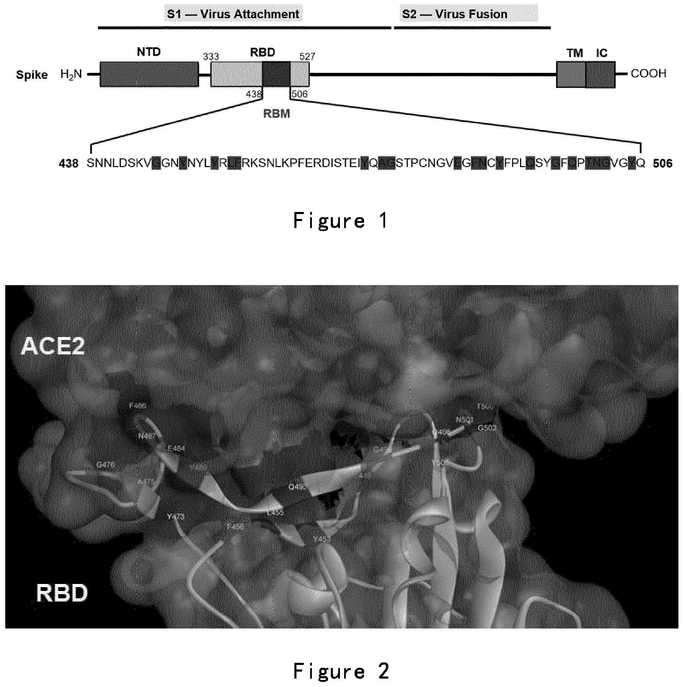

The S protein is a major structural protein of the coronavirus SARS-CoV-2, the schematic diagram of its structure is shown in Figure 1, wherein, RBD is responsible for bonding to the human ACE2 receptor, and the RBM region comprises a motif that binds to human ACE2. A amino acid sequence of a typical S protein is shown in SEQ ID No: 13.

























 (SEQ ID No: 13)

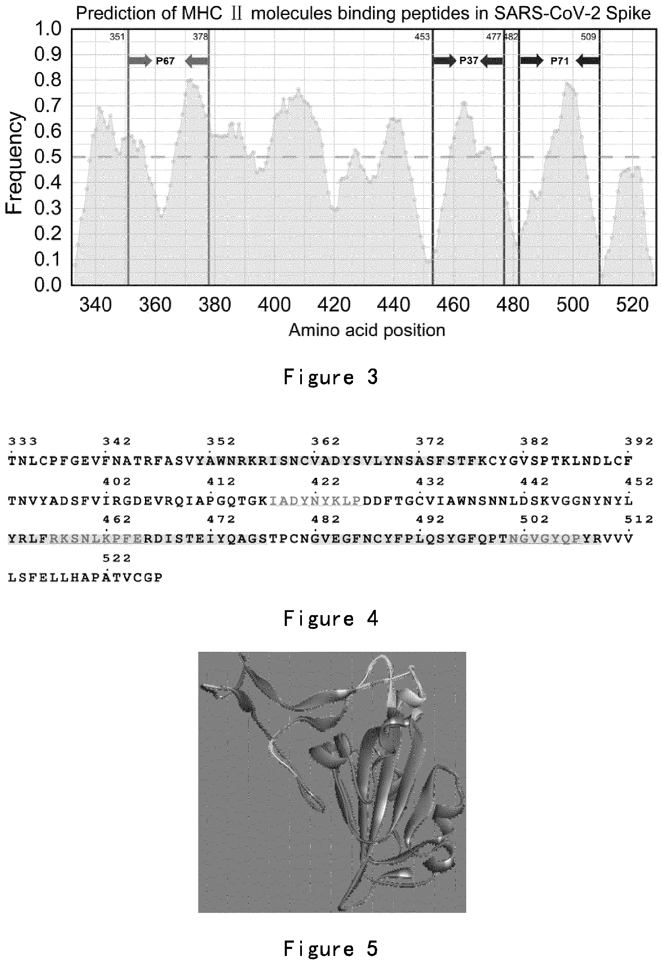

The RBD region of the coronavirus SARS-CoV-2 is located at positions 333-527 of the S protein, and a representative amino acid sequence is shown in SEQ ID No: 13 positions 333-527 or shown in Figure 4.
>RBD(333-527)




 (SEQ ID No: 13

positions 333-527)

The RBM region of the coronavirus SARS-CoV-2 is located at positions 438-506 of the S protein, and a representative amino acid sequence is shown in SEQ ID No: 13 positions 438-506 or shown in Figure 1.
>RBM(438-506)

SNNLDSKVGGNYNYLYRLFRKSNLKPFERDISTEIYQAGSTPCNGVEGF
NCYFPLQSYGFQPTNGVGYQ (SEQ ID No: 13 positions 438-506)

It should be understood that in the present invention, the S protein, RBD region and RBM region all include wild type and mutant type.

### Vaccine peptide

In the present invention, "epitope peptide of the present invention", "vaccine polypeptide of the present invention", and "polypeptide of the present invention" can be used interchangeably, and refer to the vaccine polypeptide described in the first aspect of the present invention, especially polypeptides having the structure of Formula I. It should be understood that the term includes not only one kind of vaccine polypeptide of the present invention, but also peptide set (or peptide combinations) formed by multiple vaccine polypeptides of the present invention.

In the present invention, vaccine polypeptides also include other forms, such as pharmaceutically acceptable salts, conjugates, or fusion proteins. Preferably, the vaccine polypeptide of the present invention is a conjugate (coupled peptide) formed by coupling with a TLR7 agonist, especially a coupled peptide coupled with one or more TLR7 agonists, for example, conjugates shown in Table B.

In addition, in the present invention, the preferred vaccine polypeptide has the structure shown in Formula II:

X1-X-X2 (II)

(a) X is a core fragment, wherein the sequence of the core fragment is selected from one or more of SEQ ID NO:1-12 (see Table A);
(b) each X1 and X2 is independently none, 1, 2, or 3 amino acids, and the total number of amino acids of X1 and X2 is <_ 4, preferably 3, 2, 1, and more preferably 0 or 1;
wherein, X1 and X, and X and X2, are connected by peptide bonds, peptide linkers (such as a flexible linker consisting of 1-15 amino acids) or other linkers.

In the present invention, the core fragment or vaccine polypeptide inculdes a derivative polypeptide formed by one or more (e.g. 1-5,preferably 1-3) amino acids addition, one or more(e.g 1-5,preferably 1-3) amino acids substitution, or 1-3 amino acids deletion to any one of sequence shown in SEQ ID No: 1-12, and the derivative polypeptide has the same function as the original polypeptide before derivatization.

Preferably, the core fragment or vaccine polypeptide includes 1-3 amino acids addition(preferably the N-terminal or C-terminal addition), and/or 1-2 amino acids substitution(preferably conservative amino acid substitution) to any one of sequence shown in SEQ ID No: 1-12, and still has the same function as the original polypeptide before derivatization.

Preferably, the conservative amino acid substitution is performed according to Table C.

**Table C**

| Initial residue | Representative substitutions | Preferred substitution |
|---|---|---|
| Ala (A) | Val; Leu; Ile | Val |
| Arg (R) | Lvs; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

As used herein, the peptide set described by the term "peptide set" consists of at least two kinds of vaccine polypeptides of the present invention or derivative polypeptides thereof.

Preferably, the peptide set of the present invention comprises at least 2, 3, 4, 5, 6, 7, 8, 9, 10, 11, or 12 kinds of vaccine polypeptides or derivative polypeptides thereof (including coupled peptides) selected from the first aspect of the present invention; more preferably, the peptide set comprises at least 2, 3, 4, 5, 6, 7, 8, 9, 10, 11, or 12 kinds of vaccine polypeptide selected from SEQ ID NO.: 1-12 or derivative polypeptides thereof. In addition, the peptide set may also include other antigenic peptides or proteins of coronavirus SARS-CoV-2 except SEQ ID NO.: 1-12.

As used herein, "separated" refers to the separation of a substance from its original environment (if it is a natural substance, the original environment is the natural environment). For example, the polypeptide in living cells in the natural state is not separated and purified, but the same polypeptide is separated and purified if it is separated from other substances that exist in the natural state.

As used herein, "separated peptide" means that the polypeptide of the present invention is substantially free of other proteins, lipids, carbohydrates or other substances naturally associated therewith. Those skilled in the art can use standard protein purification techniques to purify the polypeptide of the present invention. The substantially purified polypeptide (fusion protein) can produce a single main band on a non-reducing polyacrylamide gel.

The polypeptide of the present invention may be a recombinant polypeptide or a synthetic polypeptide, preferably a synthetic polypeptide.

In the present invention, when the sequence of the vaccine polypeptide is short (such as ≤ 70aa, more preferably ≤ 60aa), the relevant peptide sequence may be directly artificially synthesized by chemical methods.

When the sequence of the vaccine polypeptide is long or the vaccine polypeptide is provided in the form of a fusion protein, the recombinant method may be used to obtain the related peptide sequence in large quantities. This usually involves cloning the coding sequence of the antigenic polypeptide or its fusion protein into a vector, and then transferring it into cells, and then separating the relevant antigenic polypeptide or fusion protein from the proliferated host cell by conventional methods.

### Pharmaceutical composition and mode of administration

The invention also provides a pharmaceutical composition. The pharmaceutical composition of the present invention can be therapeutic or prophylactic (e.g. vaccine). The pharmaceutical composition of the present invention includes an effective amount of the vaccine polypeptide or peptide set of the present invention, or immune cells activated by the vaccine polypeptide (e.g., dendritic cells sensitized by the vaccine polypeptide of the present invention or T cells induced by the dendritic cells ), and at least one pharmaceutically acceptable carrier, diluent or excipient.

In another preferred embodiment, the related diseases caused by the novel coronavirus SARS-CoV-2 are selected from group consisting of: respiratory tract infection, pneumonia and its complications, and combinations thereof.

In the present invention, these (vaccine) compositions comprise immune antigens (including the vaccine polypeptides, peptide set or derivatives thereof of the present invention), and are usually combined with "pharmaceutically acceptable carriers", including any carrier that itself does not induce the production of any antibody that is harmful to the individual receiving the composition. Examples of suitable carriers include (but are not limited to) proteins, lipid aggregates (such as oil droplets or liposomes) and so on. These carriers are well known to those ordinary skilled in the art. In addition, these carriers may function as immunostimulants ("adjuvants").

In addition, the (vaccine) composition of the present invention may also comprises additional adjuvants. Representative vaccine adjuvants include (but are not limited to) the following types: inorganic adjuvants, e.g., aluminum hydroxide, alum, etc.; synthetic adjuvants, e.g., synthetic double-stranded polynucleotides (double-stranded polyadenosine acid, uridine acid), levamisole, isopinosine, etc.; oil agents, e.g., Freund's adjuvant, peanut oil emulsification adjuvant, mineral oil, vegetable oil, etc..

Generally, the vaccine composition or immunogenic composition may be made into an injectable agent, for example, a liquid solution or suspension; it may also be made into a solid form suitable for being formulated into a solution or suspension or a liquid excipient before injection. The preparation can also be emulsified or encapsulated in liposomes to enhance the adjuvant effect.

The composition may be made into a unit or multiple dosage form. Each dosage form comprises a predetermined amount of active substance calculated to produce the desired therapeutic effect, and suitable pharmaceutical excipients.

The formulated pharmaceutical composition may be administered by conventional routes, including (but not limited to): intravenous, intramuscular, intraperitoneal, subcutaneous, intradermal, oral, or topical administration.

When using a (vaccine) composition, a safe and effective amount of the vaccine polypeptide or peptide set of the present invention is administered to a human, wherein the safe and effective amount is usually at least about 1 ug peptide/kg body weight, and in most cases not more than about 8 mg peptide/kg body weight, preferably the dose is about 1 ug-1 mg peptide/kg body weight. Of course, the specific dose should also consider factors such as the administration route and the patient's health status, which are all within the skill range of a skilled physician.

The main advantages of the present invention include:
(a) The vaccine polypeptides (including coupled polypeptides) used in the present invention can produce neutralizing antibodies against S protein RBD (receptor binding domain) in the body of mammals such as primates, and the neutralizing antibodies can block the binding of RBD to ACE2.
(b) Compared with the unoptimized polypeptide, the coupled peptides formed by coupling the optimized polypeptide sequence of the present invention with the TLR7 small molecule agonist have an optimized structure and can produce cellular immunity and humoral immunity against SARS-CoV-2 virus efficiently.

The present invention is further explained below in conjunction with specific example. It should be understood that these embodiments are only for illustrating the present invention and not intend to limit the scope of the present invention. The conditions of the experimental methods not specifically indicated in the following examples are usually in accordance with conventional conditions as described in Sambrook et al., Molecular Cloning: A Laboratory Manual (New York: Cold Spring Harbor Laboratory Press, 1989), or according to the conditions recommended by the manufacturer. Unless otherwise stated, percentages and parts are calculated by weight.

### Example 1 Antigen peptide screen based on sequence and structure analysis of S protein RBD

In this example, the inventors used the RBD region in the S protein of SARS-CoV-2 that interacts with human ACE2 as the analysis object, and determined the key interaction sites in the RBD, as shown in Figure 1 and Figure 2.

The inventors used software to predict and analyze the CD8+T cell epitopes in the RBD sequence; used Allele Frequency Net Database to statistical analysis and obtain the main HLA class II molecular allele types of the world population; further analyzed and predicted the HLA class II molecular binding peptides in the RBD sequence, which were used as predicted CD4+ T cell epitopes; used BepiPred and Discotope software to predict linear and conformational B cell epitopes in the RBD sequence; used Uniprot database to obtain glycosylation modification sites in the RBD region; and used ProtParam software to predict the physical and chemical properties of candidate peptides.

Based on the above analysis results and information, the inventors finally screened 12 polypeptides with the following amino acid sequences:

**Table A Antigenic peptides**

| Peptide ID | Sequence | Length (aa) | SEQ ID No: |
|---|---|---|---|
| P-33 | YNYLYRLFRKSNLKPFERDISTEIY | 25aa | 1 |
| P-37 | YRLFRKSNLKPFERDISTEIYQAGS | 25aa | 2 |
| P-41 | KRSFIEDLLFNKVTLADAGFIKQYG | 25aa | 3 |
| P-67 | YAWNRKRISNCVADYSVLYNSASFS TFK | 28aa | 4 |
| P-67-F1 | CYAWNRKRISN | 11aa | 5 |
| P-67-F2 | CVADYSVLYNSASFSTFK | 18aa | 6 |
| P-71 | GVEGFNCYFPLQSYGFQPTNGVGY QPYR | 28aa | 7 |
| P-73 | FQPTNGVGYQPYRVVVLSFELLHAP ATV | 28aa | 8 |
| P-79 | ISNCVADYSVLYNSASFSTFKC | 22aa | 9 |
| P-85 | DYSVLYNSASFSTFKCYGVSPT | 22aa | 10 |
| P-86 | TEIYQAGSTPCNGVEGFNCYFP | 22aa | 11 |
| LY54 | LFRKSNLKPFERDISTEIYQAGSTPC NGVEGFNCYFPLQSYGFQPTNGVG YQPY | 54aa | 12 |

Furthermore, according to the comprehensive characteristics, P-37, P-67 (including P-67-F1 and P-67-F2), P-71 and LY54 were determined.

The allelic types of HLA class I molecules corresponding to the CD8+ T cell epitopes contained in polypeptides P-37, P-67 and P-71 are shown in Table 1, suggesting that those three polypeptides have better potential to stimulate the antiviral response of killer T cells.

**Table 1 HLA class I allele types corresponding to CTL epitopes contained in some polypeptides**

| **Peptide ID** | **Position** | **Sequence** | **Potential HLA I Alleles** |
|---|---|---|---|
| P-37 | 453-477 | YRLFRKSNLKPFERDISTEIYQAGS | HLA-A^{∗}03:01, HLA-B^{∗}08:01, HLA-B^{∗}27:05, HLA-B^{∗}39:01, HLA-B^{∗}40:01 |
| P-67 | 351-378 | YAWNRKRISNCVADYSVLYNSASFSTFK | HLA-A^{∗}01:01, HLA-A^{∗}03:01, HLA-A^{∗}24:02, HLA-A^{∗}26:01, HLA-B^{∗}15:01 |
| P-71 | 482-509 | GVEGFNCYFPLQSYGFQPTNGVGYQPYR | HLA-A^{∗}24:02, HLA-B^{∗}39:01 |

The binding affinity of the three polypeptides (P-37, P-67 and P-71) to 24 major HLA class II typing molecules in the population are shown in Table 2 and Figure 3. This suggests that the three peptides can be presented by the main HLA II molecules in the population to stimulate a wide range of CD4 + T cell effects and promote the potential of B cells to produce antiviral antibodies.

**Table 2 Prediction analysis of binding affinity of polypeptides P-37, P-67 and P-71 to 24 major HLA class I allele typing molecules in the population**

| **HLAIIAlleles** | **P-37** | **P-67** | **P-71** | **HLAIIAlleles** | **P-37** | **P-67** | **P-71** |
|---|---|---|---|---|---|---|---|
| HLA-DRB1^{∗}07:01 | 225.1 | 174.47 | 119.18 | I-ILA DRB 1 * 14 01 | 453.15 | 303.75 | 1167.69 |
| HLA-DRB1^{∗}03:01 | 286.82 | 23039 | 682896 | HLA-DRB1^{∗}01:02 | 1269.86 | 815.43 | 841.65 |
| HLA-DRB1^{∗}15:01 | 392.67 | 163.05 | 266.07 | HLA-DRB1^{∗}15:03 | 826.81 | 372.66 | 397.31 |
| HLA-DRB1^{∗}11:01 | 198.63 | 402.78 | 710.44 | HLA-DRB1^{∗}12:01 | 1164.88 | 532.98 | 1374.95 |
| HLA-DRB1^{∗}01:01 | 247.37 | 72.84 | 69.27 | HLA-DRB1^{∗}04:05 | 382 | 145.29 | 299.53 |
| HLA-DRB1^{∗}13:02 | 186.59 | 67.24 | 805.85 | HLA-DRB1^{∗}10:01 | 195.67 | 47.77 | 42.95 |
| HLA-DRB1^{∗}13:01 | 277.36 | 241.6 | 1321.87 | HLA-DRB1^{∗}04:07 | 846.13 | 327.22 | 1245.31 |
| HLA-DRB1^{∗}04:01 | 275.96 | 127.44 | 332.64 | HLA-DRB1^{∗}04:03 | 1429.01 | 336.16 | 1716_56 |
| HLA-DRB1^{∗}11:04 | 382.03 | 868.22 | 1557.92 | HLA-DRB3^{∗}01:01 | 55.22 | 139.35 | 1873.46 |
| HLA-DRB1^{∗}04:04 | 681.78 | 154.24 | 559.56 | HLA-DRB3^{∗}02:02 | 248.29 | 66.73 | 817.85 |
| HLA-DRB1^{∗}15:02 | 335.07 | 100.46 | 326.86 | HLA-DRB4^{∗}01:01 | 285.49 | 277.78 | 1086.17 |
| HLA-DRB1^{∗}09:01 | 367.16 | 159_26 | 109.83 | HLA-DRB5^{∗}01:01 | 145.79 | 319.83 | 299.8 |

Further analysis showed that the three polypeptides (P-37, P-67 and P-71) are good overlapped with the linear and conformational B cell epitopes of the RBD (Figure 4 and Figure 5), suggesting that B cells immune response can be effectively activated.

In addition, the three polypeptides (P-37, P-67 and P-71) have no glycosylation modification sites and have suitable physical and chemical properties.

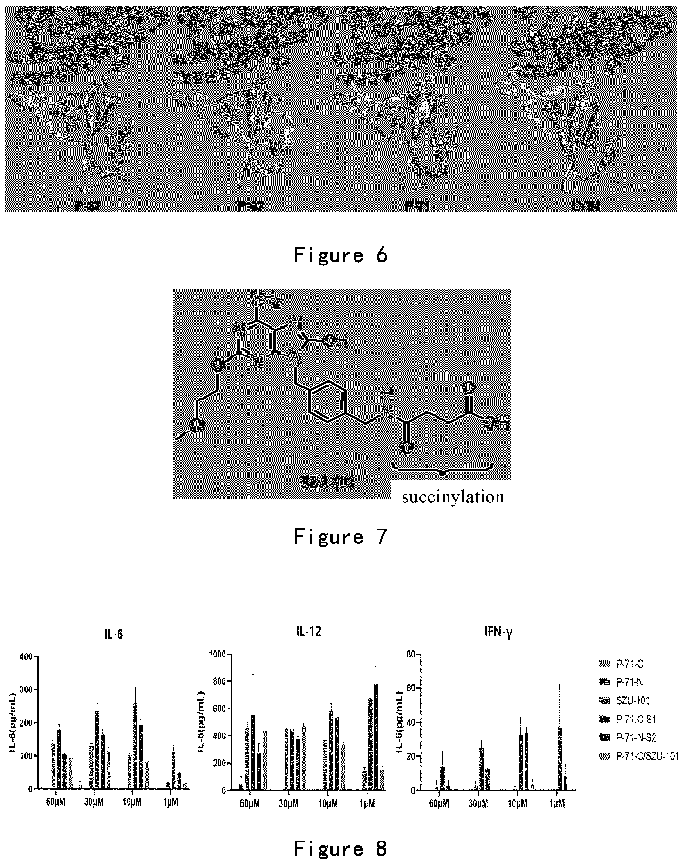

LY54 is a long peptide comprising both P-37 and P-71 epitopes, which stretchs over the interface between RBD and ACE2, as shown in Figure 6.

### Example 2 Preparation of polypeptides

In this example, a peptide synthesizer was used to prepare polypeptides P-37, P-67, P-71 and LY54.

| Peptide | Sequence | Remarks |
|---|---|---|
| P-37 peptide | CYRLFRKSNLKPFERDISTEIYQA GS | SEQ ID No: 2, a C is added at the N-terminal |
| P-67-F1 peptide | CYAWNRKRISN | SEQ ID No: 5 |
| P-67-F2 peptide | CVADYSVLYNSASFSTFK | SEQ ID No: 6 |
| P-71-C peptide | GVEGFNCYFPLQSYGFQPTNGVG YQPYRK | SEQ ID No: 7, a K is added at the C-terminal |
| P-71-N peptide | KGVEGFNCYFPLQSYGFQPTNGV GYQPYR | SEQ ID No: 7, a K is added at the N-terminal |
| LY54 peptide | LFRKSNLKPFERDISTEIYQAGSTP CNGVEGFNCYFPLQSYGFQPTNG VGYQPY | SEQ ID No: 12 |

### Example 3 Preparation of coupled peptides

Artificially synthesized polypeptides P-37, P-67 (including P-67-F1 and P-67-F2), P-71 and LY54, etc., were coupled with TLR7 small molecule agonist to form corresponding coupled peptide.

The molecular structure of TLR7 small molecule agonist for coupled polypeptides is as follows:

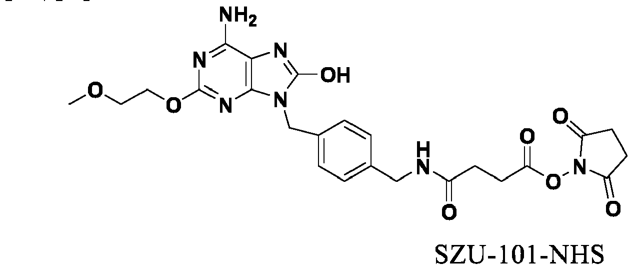

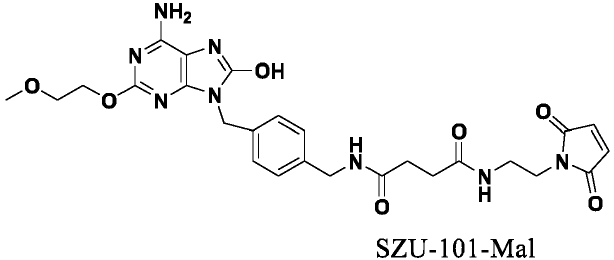

### 3.1 Preparation of coupled peptide P-37-S2

Preparation of conjugate of P-37 peptide (CYRLFRKSNLKPFERDISTEIYQAGS) and TLR7 small molecule agonist. 18 mg of P-37 peptide was weighted and dissolved into a mixed solution of 270µL H₂O and 180µL DMF, then 4.88 mg of SZU-101-Mal and NaHCO₃ with a final concentration of 30 mM were added at the same time. After reacted at room temperature for 1 hour, the products were separated by a C18 preparation column to obtain SZU-101 and P-37 peptide coupled product P-37-S2 total 15.5mg, HRMS [M+5H]⁵⁺ 740.9976.

### 3.2 Preparation of coupled peptide P-67-F1-S2

Preparation of conjugate of P-67-F1 peptide (CYAWNRKRISN) and TLR7 small molecule agonist. 16.5mg of P-67-F1 peptide was weighted and dissolved into 330µL H₂O, then 984µL solution of SZU-101-Mal in DMF (20 mg of SZU-101-Mal was weighted and dissolved into 2mL DMF in advance to form a 10mg/mL stock solution) was added, while adding NaHCO₃ with a final concentration of 30mM. After reacted at room temperature for 1 hour, the product was separated by C18 preparation column to obtain SZU-101 and P-67-F1 peptide coupled product P-67-F1-S2 A total 9.3mg, HRMS [M+4H]⁴⁺ 494.2456.

### 3.3 Preparation of coupled peptide P-67-F2-S2

Preparation of conjugate of P-67-F2 peptide (CVADYSVLYNSASFSTFK) and TLR7 small molecule agonist. 20 mg of P-67-F2 peptide was weighted and dissolved into 2 mL of 50% acetonitrile aqueous solution containing 0.1% trifluoroacetic acid (TFA), then 675µE solution of SZU-101-Mal in DMF (20 mg of SZU- 101-Mal was weighted and dissolved into 2mL DMF in advance to form a 10mg/mL stock solution) was added, while adding NaHCO₃ with a final concentration of 100mM. After reacted at room temperature for 1 hour, the products was separated by C18 preparation column to obtain SZU-101 and P-67-The F2-S2 peptide coupled product P-67-F2-S2 totals 13.5mg, HRMS [M+3H]³⁺ 861.4077.

### 3.4 Preparation of coupled peptide P-71-S2

Preparation of conjugate of P-71-C peptide (GVEGFNCYFPLQSYGFQPTNGVGYQPYRK) and TLR7 small molecule agonist. 20mg of P-71-C peptide was weighted and dissolved into 400µL H₂O, then 461µL solution of SZU-101-Mal in DMF (20mg SZU-101-Mal was weighted and dissolved into 2mL DMF in advance to form a 10mg/mL stock solution), while adding NaHCO₃ with a final concentration of 30mM. After reacted at room temperature for 1 hour, the product was separated by C18 preparation column to obtain SZU-101 and P-71-C peptides coupled product P-71-S2 total 20mg, HRMS [M+4H]⁴⁺ 971.4554.

### 3.4 Preparation of coupled peptide P-71-C-S1

Preparation of conjugate of P-71-C peptide (GVEGFNCYFPLQSYGFQPTNGVGYQPYRK) and TLR7 small molecule agonist(2). 18mg of P-71-C peptide was weighted and dissolved into 360µL H₂O, then 850µL solution of SZU-101-NHS in DMF (dissolved in DMF in advance to form a 20mg/mL stock solution), while adding NaHCO₃ with a final concentration of 20mM. After reacted at room temperature for 1 hour, a product of coupled 3 SZU-101 small molecules was formed. Then DTT at a final concentration of 5mM was added, and reacted at 37°C for 1 hour to form a product of coupled 2 SZU-101. The product was separated by C18 preparation column to obtain SZU-101 and P-71-C peptides coupled product P-71-C-S1 total 12 mg, HRMS [M+4H]⁴⁺ 1042.9789.

### 3.5 Preparation of coupled peptide P-71-N-S1

Preparation of conjugate of P-71-N peptide (KGVEGFNCYFPLQSYGFQPTNGVGYQPYR) and TLR7 small molecule agonist. 22 mg of P-71-N peptide was weighted and dissolved into 440µL H₂O, 1000µL solution of SZU-101-NHS in DMF (dissolved in DMF in advance to form a 20mg/mL stock solution) was added, while adding NaHCO₃ with a final concentration of 20mM. After reacted at room temperature for 1 hour, a product of coupled 3 SZU-101 small molecules was formed. Then DTT at a final concentration of SMm was added and reacted at 37°C for 1 hour to form a product of coupled 2 SZU-101. The product was separated by C18 preparation column to obtain SZU-101 and P-71-N peptides coupled product P-71-N-S1 total 19 mg, HRMS [M+4H]⁴⁺ 1042.9789.

### 3.6 Preparation of coupled peptide LY54-S1

Preparation of conjugate of LY54 peptide (LFRKSNLKPFERDISTEIYQAGSTPCNGVEGFNCYFPLQSYGFQPTNGVGYQP Y) and TLR7 small molecule agonist. 10mg of P-71-C peptide was weighted and dissolved into 200µL H₂O, then 442µL solution of SZU-101-Mal in DMF (20mg SZU-101-Mal was weighted and dissolving into 2mL DMF in advance to form a 20mg/mL stock solution), while adding NaHCO₃ with a final concentration of 20mM. After reacted at room temperature for 1 hour, a product of coupled 5 SZU-101 small molecules is formed. Then DTT at a final concentration of 5mM was added and reacted at 37°C for 1 hour to form a product of coupled 3 SZU-101. The product was separated by C18 preparation column to obtain SZU-101 and LY54 peptides coupled product LY54-S1 total 7.2mg, HRMS [M+4H]⁺⁶⁺ 1035.0771.

### Example 4 In vitro mouse splenic lymphocyte proliferation and cytokine release experiments

The P-37, P-67, P-71 and other polypeptides prepared in example 3, as well as the coupled peptides formed after coupled with the TLR7 agonist SZU-101, were added into the culture medium of the primary spleen lymphocyte isolated and prepared from the spleen of BALB/c mice, and four dose groups of 1µM, 10µM, 30µM, and 60µM were contained. The cytokines IL-6, IL-12 and IFN-γ were tested after 24 hours stimulation, and cell proliferation was tested after 72 hours stimulation.

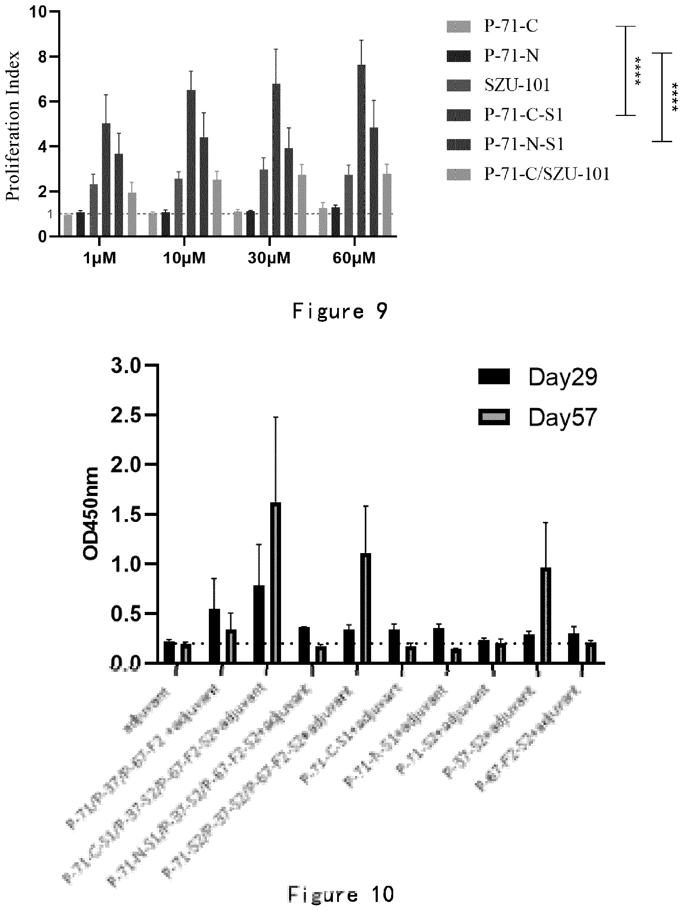

Taking P-71 and its corresponding coupled peptide as an example, as shown in Figure 8 and Figure 9, the results show that:
(1) The polypeptides alone cannot or weakly cause the increase of cytokine release of splenic lymphocytes, and the coupled peptide after coupled with SZU-101 can effectively trigger the increase of cytokine release of splenic lymphocytes, which suggests that the immune response induced by the vaccine polypeptide can be significantly improved after coupled with TLR7 agonists;
(2) The polypeptides alone cannot or weakly cause the proliferation of splenic lymphocytes, and the coupled peptide after coupled with SZU-101 can effectively trigger the proliferation of splenic lymphocytes, which suggests that the immune response induced by vaccine polypeptides can be significantly improved after coupled with TLR7 agonists.

### Example 5 Mouse immunization experiment of coupled polypeptide vaccine

The P-37, P-67, P-71 and other polypeptides prepared in example 3, as well as the coupled peptides formed after coupling with the TLR7 agonist SZU-101 are used alone or in combination. When used in combination, the mixing ratio is 1: 1:1 (mass ratio), prepared with aluminum hydroxide as an adjuvant, and injected into the abdominal cavity to immunize BALB/c mice.

The serums were collected at 29 days and 57 days after the first immunization, and the antibodies against the S protein RBD of SARS-CoV-2 in the serum (1:100-fold dilution) were detected by the bridge-ELISA method.

As shown in Figure 10, the results show that:
(1) In the mixed peptides containing P-71-C-S1 or P-71-S2 + adjuvant group, antibodies against RBD can be significantly produced;
(2) In the conjugate P-37-S2 alone + adjuvant group, antibodies against RBD can be significantly produced;
(3) Although the mixed peptides not coupled with SZU-101 can induce the production of antibodies against RBD, the amount of antibodies produced is lower than that of the mixed peptides group containing the coupled peptides, which suggests that the immune response induced by vaccine polypeptides can be significantly improved after coupled with TLR7 agonists.

### Example 6 Cynomolgus monkey immune experiment of the coupled polypeptide vaccine

In this example, the immune effect of the coupled polypeptide vaccine against SARS-CoV-2 was further verified in cynomolgus monkeys.
(a) a Mixed coupled peptides of P-37, P-67-F2, P-71 coupled with SZU-101(P-71-C-S1/P-71-N-S1/P-71-S2/P-37-S2/P-67-F2-S2), (b) LY54, (c) LY54-S1, were prepared into immune preparations with TiterMax adjuvant, respectively, and injected subcutaneously in Cynomolgus monkeys at multiple points for immunization. 14 days after the second immunization, the antibody titer was measured by Bridging-ELISA method, and the ability of antiserum to block the binding of RBD and ACE2 was measured.

The neutralizing antibody was detected by a competitive ELISA method, and the specific determination method is as follows: an ELISA plate was coated overnight with 10 µg/mL ACE2 , and then blocked for use. The anti-peptide serum diluted in different degrees with sample dilution buffer(1:128, 1:64, 1:32, 1:16, 1:8, and 1:4) , then the diluted anti-serum in different degrees were incubated with Bio-RBD of 12 µg/mL at 37°C for 1 hour. Then 100 µL of the reaction mixture was added into the wells of the blocked ACE2 coated ELISA plate, and incubated at 37°C for 1 hour. Then the plate was washed, HRP-Streptavidin A diluted in 1:10000 was added and incubated at 37°C for 1 hour. After washed the plate, TMB was added for color development, and the plate was read at 450nm wavelength after termination.

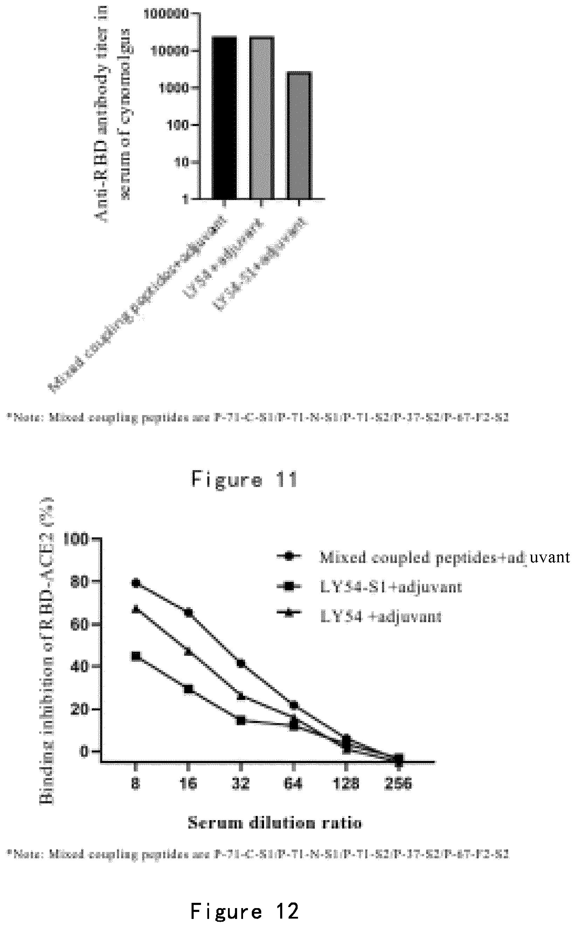

The results show that all animals produced antibodies against RBD, and the antibody titers are all high (Figure 11).

In addition, all of the antiserum produced by the mixed coupled peptides, LY54 and LY54-S1 have the ability to block the binding of RBD to ACE2 (Figure 12), that is, they have the effect of blocking the infection of SARS-CoV-2 virus.

### Example 7 In vitro binding experiment of polypeptide and ACE2

In this example, the polypeptides P-37, P-71, and LY54 whose spatial structure are located on the interface between RBD and ACE2 are labeled with biotin, and the binding ability of the above-mentioned polypeptides to ACE2 are detected by ELISA.

The specific determination method is as follows: the ELISA plate was coated overnight with 10ug/mL of ACE2, blocked, and then biotin-labeled polypeptides in different concentration (1, 0.5 and 0.25 µg/mL) is added. After incubated at 37°C for 1.5 hours, HRP-Streptavidin A diluted in 1: 5000 was added, and incubated at 37°C for 1 hour. After washed the plate, TMB was added for color development, and the plate was read at 450nm wavelength after termination.

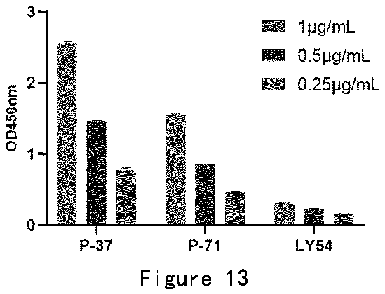

The results show that the peptides P-37, P-71 and LY54 have a strong binding ability with ACE2 (Figure 13), suggesting that the peptides P-37, P-71 and LY54 themselves have potential blocking effects.

### Discussion

Polypeptide vaccine is one or more epitope fragments selected from highly immunogenic proteins for immunization. Due to the short amino acid chain of polypeptide vaccine, and thanks to the maturity of peptide synthesis technology, it facilitates rapid and large-scale *in vitro* synthesis and purification, and it is easy to ensure the purity and repeatability of each batch of products.

Meanwhile, computer-assisted vaccine design can quickly respond to emergent public health problems caused by novel viruses, and predict and screen suitable candidate peptides for vaccine development.

In addition, compared with other types of vaccines, polypeptide vaccines have clear epitopes, good stability, high purity and better safety. However, as the amino acid chain length is short, usually about 10-30 amino acids, polypeptide vaccines also face the problem of low immunogenicity, and often need to be modified or supplemented with adjuvants to produce a better immune response.

The design of polypeptide vaccines requires the help of computer-assisted vaccine design technology, which often requires comprehensive analysis of T/B cell epitopes, structure and modification information of the target protein. Wherein, CD8+T cell epitopes can activate killer T cells to exert antiviral effects, CD4+T cell epitopes mainly activate helper T cells to activate B cells to produce antiviral antibodies, and linear and conformational B cell epitopes can directly activate B cells to produce antibodies.

SARS-CoV-2 invades host cells by binding to human Angiotensin-convertion enzyme 2 (ACE2) protein through its surface Spike glycoprotein (S protein). Therefore, S protein is the preferred target protein for COVID-19 vaccine design, in the hope of inducing the production of neutralizing antibodies that can block virus invasion in the body. However, currently known vaccines are difficult to effectively trigger the body to produce an immune response against the coronavirus SARS-CoV-2.

Therefore, activating high-efficiency antigen-presenting cells against SARS-CoV-2 is the key to the development of SARS-CoV-2 vaccines.

Through research, the inventors unexpectedly discovered that the vaccine polypeptides that based on the screened and sequence optimized antigenic polypeptides of the S protein of coronavirus SARS-CoV-2 and further coupled with TLR7 small molecule agonist, may effectively induce the production of antiviral antibodies which against the RBD region of the S protein and have a blocking effect in animal organism including primates. In addition, the vaccine polypeptide coupled with TLR7 small molecule agonist SZU-101 can efficiently activate antigen presenting cells, thus overcoming the problems of low polypeptide immunogenicity and immune escape of viruses.

Therefore, the coupled peptides of the present invention can be used to develop a novel coronavirus polypeptide vaccine. By inducing cellular immunity and humoral immunity in the human body, it can be used to prevent and treat coronavirus SARS-CoV-2 infection and related diseases, including corona virus disease 2019 (COVID-19).

In addition, the strategy of the present inventors that coupled TLR7 agonists with specific antigenic polypeptides to form coupling polypeptides (especially coupled with TLR7 small molecule agonists SZU-101, such as S1 and/or S2 structure coupled polypeptides) is also suitable for the development of drugs or vaccines for the prevention and treatment of other single-stranded RNA virus (ssRNA virus) infections or related diseases, especially for the development of vaccines against various coronaviruses, including the known coronavirus(SARS, MERS, etc.) and unknown coronavirus.

All references mentioned in the present application are incorporated by reference herein, as though individually incorporated by reference. In addition, it should be understood that after reading the above teaching content of the present invention, various changes or modifications may be made by those skilled in the art, and these equivalents also fall within the scope as defined by the appended claims of the present application.

## Claims

1. A vaccine polypeptide of the novel coronavirus, wherein the vaccine polypeptide has a structure of Formula I or an oligomer comprising the structure of Formula I:
Z-(J-U)n (I)
wherein,
Z is an antigenic polypeptide that has at least one T cell epitope and/or at least one B cell epitope of the novel coronavirus S protein; and, the antigenic polypeptide has an amino acid sequence derived from the RBM region of the S protein;
each U is independently a TLR7 agonist;
n is 0 or a positive integer; and
J is a chemical bond or linker.

2. The vaccine polypeptide of claim 1, wherein the vaccine polypeptide can stimulate primates and rodents to produce neutralizing antibodies that block the binding of RBD to ACE2.

3. The vaccine polypeptide of claim 1, wherein the antigenic polypeptide is selected from the group consisting of:
(a) a pollypeptide having any one of the amino acid sequence shown in SEQ ID No: 1-12; and
(b) a derivative polypeptide formed by one or more amino acids addition, one or more amino acids substitution, or 1-3 amino acids deletion to the amino acid sequence of the polypeptide in (a), and the derivative polypeptide has the same function as the original polypeptide before derivatization.

4. The vaccine polypeptide of claim 1, wherein the structure of the antigenic polypeptide is as shown in Formula II:
X1-X-X2 (II),
Wherein,
(a) X is a core fragment, wherein the sequence of the core fragment is selected from one or more of SEQ ID NO:1-12 (see Table A);
(b) each X1 and X2 is independently none, 1, 2, or 3 amino acids, and the total number of amino acids of X1 and X2 is ≤ 4, preferably 3, 2, 1, and more preferably 0 or 1; and
(c) "-" represents peptide bond, peptide linker, or other linker (that is, X1 and X and/or X and X2, are connected by peptide bonds, peptide linkers (such as a flexible linker consisting of 1-15 amino acids) or other linkers).

5. The vaccine polypeptide of claim 1, wherein the antigenic polypeptide is selected from Table A:
**Table A Antigenic peptides**
| Peptide ID | Sequence | Length (aa) | SEQ ID No: |
|---|---|---|---|
| P-33 | YNYLYRLFRKSNLKPFERDISTEIY | 25aa | 1 |
| P-37 | YRLFRKSNLKPFERDISTEIYQAGS | 25aa | 2 |
| P-41 | KRSFIEDLLFNKVTLADAGFIKQYG | 25aa | 3 |
| P-67 | YAWNRKRISNCVADYSVLYNSASFSTF K | 28aa | 4 |
| P-67-F1 | CYAWNRKRISN | 11aa | 5 |
| P-67-F2 | CVADYSVLYNSASFSTFK | 18aa | 6 |
| P-71 | GVEGFNCYFPLQSYGFQPTNGVGYQP YR FQPTNGVGYQPYRVVVLSFELLHAPAT V | 28aa | 7 |
| P-73 | | 28aa | 8 |
| P-79 | ISNCVADYSVLYNSASFSTFKC | 22aa | 9 |
| P-85 | DYSVLYNSASFSTFKCYGVSPT | 22aa | 10 |
| P-86 | TEIYQAGSTPCNGVEGFNCYFP | 22aa | 11 |
| LY54 | LFRKSNLKPFERDISTEIYQAGSTPCNG VEGFNCYFPLQSYGFQPTNGVGYQPY | 54aa | 12. |

6. The vaccine polypeptide of claim 1, wherein the TLR7 agonist comprises: SZU-101 :

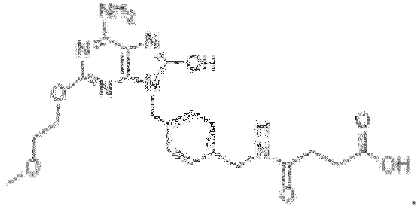

7. The vaccine polypeptide of claim 6. wherein the SZU-101 is attached to the amino group of ths antigenic polypeptide and forms a structure shown in S1: 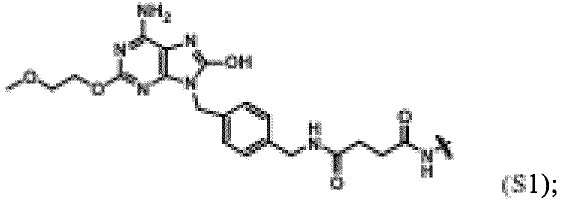 or
The SZU-101 is attached to the sulfhydryl group of the antigenic polypeptide and forms the structure shown in S2:

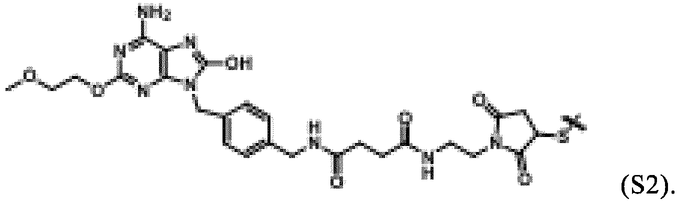

8. The vaccine polypeptide of claim 7, wherein the U is site-specifically linked to Z;
Preferably, the vaccine polypeptide is selected from the group of coupled peptides consisting of:
(S1)-GVEGFNCYFPLQSYGFQPTNGVGYQPYRK-(S1) (SEQ ID No: 14)
wherein, SZU-101 is attached to the N-terminal amino group of G and the side chain amino group of K in the amino acid sequence through the S 1 structure;
(S1)₂-KGVEGFNCYFPLQSYGFQPTNGVGYQPYR (SEQ ID No: 15)
wherein, SZU-101 is attached to the N-terminal and side chain amino group of K in the amino acid sequence through the S1 structure; 
(S1)-LFRK(-S1)SNLK(-S1)PFERDISTEIYQAGSTPCNGVEGFNCYFPLQSY
GFQPTNGVGYQPY(SEQ ID No: 12)
wherein, SZU-101 is attached to the N-terminal amino group of L and the side chain amino group of K in the amino acid sequence through the S1 structure;
GVEGFNC(-S2)YFPLQSYGFQPTNGVGYQPYRK (SEQ ID No: 14)
wherein, SZU-101 is attached to the sulfhydryl group of C in the amino acid sequence through the S2 structure;
(S2)-CYRLFRKSNLKPFERDISTEIYQAGS (SEQ ID No: 16)
wherein, SZU-101 is attached to the sulfhydryl group of C in the polypeptide through the S2 structure;
(S2)-CYAWNRKRISN (SEQ ID No: 5)
wherein, SZU-101 is attached to the sulfhydryl group of C in the polypeptide through the S2 structure; and
(S2)-CVADYSVLYNSASFSTFK (SEQ ID No: 6)
wherein, SZU-101 is attached to the sulfhydryl group of C in the polypeptide through the S2 structure.

9. The vaccine polypeptide of claim 5, wherein the vaccine polypeptide is selected from the group consisting of: P-37 or its conjugate with TLR7 agonist, P-67-F1 or its agonist with TLR7, P-71 or its conjugate with TLR7 agonist, LV54 or its conjugate with TLR7 agonist, and combinations thereof.

10. A separated peptide set, wherein the peptide set comprises at least two vaccine polypeptides of the novel coronavirus according to claim 1.

11. A pharmaceutical composition, wherein the pharmaceutical composition comprises the vaccine polypeptide of novel coronavirus of claim 1 or the peptide set of claim 7, and a pharmaceutically acceptable carrier.

12. A use of the novel coronavirus vaccine polypeptide of claim 1 or the peptide set of claim 10 or the pharmaceutical composition of claim 11, in the manufacture of a medicament for the prevention of coronavirus SARS-CoV-2 infection or related disease.

13. A cell preparation, wherein the cell preparation comprises (a) immune cells immuno-activiated by the novel coronavirus vaccine polypeptide of claim 1 or the peptide set of claim 10; and (b) a pharmaceutically acceptable carrier.

14. The cell preparation of claim 13, wherein the immune cells are selected from the group consisting of: dendritic cells, natural killer cells NK, lymphocytes, monocytes/macrophages, granulocytes, and combinations thereof.

15. A fusion protein, wherein the fusion protein comprises a carrier protein and the vaccine polypeptide of claim 1 fused to the fusion protein.

16. A pharmaceutical composition, comprising (a) the fusion protein of claim 15 or immune cells immuno-activated by the fusion protein; and (b) a pharmaceutically acceptable carrier.
